# EUROPÄISCHE PATENTSCHRIFT

(11) **EP 2 162 430 B1**
(45) Veröffentlichungstag und Bekanntmachung des Hinweises auf die Patenterteilung: **30.11.2016**
(21) Anmeldenummer: 08759722.5
(22) Anmeldetag: 19.05.2008
(51) Int. Cl.: C07C 319/04, C07C 321/04, C08F 36/04, C08F 2/38

(54) **MERCAPTANGEMISCH**
MERCAPTAN MIXTURE
MÉLANGE DE MERCAPTANS

(30) Priorität: 22.05.2007 DE 102007024009
(43) Veröffentlichungstag der Anmeldung: 17.03.2010
(73) Patentinhaber: ARLANXEO Deutschland GmbH, 41540 Dormagen (DE)
(72) Erfinder: OBRECHT, Werner, 47447 Moers (DE); PASK, Stephen, 41542 Dormagen (DE); KOEHLER, Wilfried, 51429 Bergisch Gladbach (DE); KLIMPEL, Michael, 67760 Gambsheim (FR)
(74) Vertreter: Wichmann, Birgid
(86) Internationale Anmeldenummer: PCT/EP2008/056093
(87) Internationale Veröffentlichungsnummer: WO 2008/142037

(56) Entgegenhaltungen:
- WO-A-2005/030710
- GB-A- 823 823
- JP-A- 7 316 126

## Beschreibung

Die Erfindung betrifft ein Gemisch enthaltend verschiedene Mercaptane, ein Verfahren zu dessen Herstellung und dessen Verwendung als Molekulargewichtsregler bei der Herstellung von synthetischen Kautschuken.

Mercaptane werden für die Molekulargewichtsregelung bei radikalischen Polymerisations-reaktionen verwendet. Dieser Einsatz ist unabhängig davon möglich, ob die Polymerisation in Masse, Suspension, Lösung oder in Emulsion durchgeführt wird.

Zur Molekulargewichtsregelung von Emulsionskautschuken auf der Basis von Monomeren wie Styrol, Butadien, Acrylnitril, (Meth)acrylsäure, Fumarsäure, Ethylacrylat, Butylacrylat, 2-Ethylhexylacrylat, Chloropren und anderen ist die Verwendung von Dodecylmercaptanen von Bedeutung.

In US-A-2,434,536 wird beschrieben, dass synthetische Kautschuke auf Basis von Diolefinen wie z.B. Butadien und gegebenenfalls weiteren copolymerisierbaren Monomeren wie z.B. Styrol, α-Methylstyrol, Vinylnaphthalin, Acrylnitril, Methacrylnitril, Methylmethacrylat, Ethylfumarat oder Methylvinylketon durch Emulsionspolymerisation in Gegenwart von aliphatischen Mercaptanen als Molekulargewichtsreglern hergestellt werden. Offenbart wird, dass diese Mercaptane mindestens 7 und bevorzugt 10 oder mehr Kohlenstoff-Atome aufweisen. Bevorzugt werden aliphatische Mercaptane mit einem mittleren Molekulargewicht von 188 bis 230 eingesetzt, welche mindestens 50% Dodecylmercaptan und den restlichen Anteil zu 100% in Form von Mercaptanen mit 10 bis 16 Kohlenstoffatomen aufweisen.

In Ullmanns Enzyklopädie der technischen Chemie, 4. Auflage, Band 13, Seite 611-612 wird allgemein beschrieben, dass das Molekulargewicht von Nitril-Butadien-Kautschuken durch den Einsatz von Alkylmercaptanen, Di- und Polysulfide oder Xanthogendisulfiden geregelt werden kann. Hierbei werden tert-Dodecylmercaptan sowie Diisopropylxanthogendisulfide als hauptsächlich eingesetzte Regler genannt.

Tert.-Dodecymercaptan wird meistens durch sauer katalysierte Addition von Schwefelwasserstoff an Olefine mit 12 Kohlenstoffen hergestellt. Als C₁₂-Olefin Ausgangsmaterial (auch als "C₁₂-Feedstock" bezeichnet) werden überwiegend Oligomerengemische auf der Basis von tetramerisiertem Propen (auch als Tetrapropen" oder "Tetrapropylen" bezeichnet), trimerisiertem Isobuten (auch als "Triisobutens" oder "Triisobutylen" bezeichnet), trimerisiertem n-Buten und dimerisiertem Hexen verwendet.

In Chimie & Industrie, Vol 90 (1963), No 4, 358-368 wird der Stand der Technik zur Herstellung von Mercaptanen bis 1962 beschrieben. Neben der Mercaptansynthese auf der Basis von Alkoholen spielt die Addition von Schwefelwasserstoff an Olefine insbesondere in den Vereinigten Staaten eine besondere Rolle. Für die katalysierte Schwefelwasserstoffaddition wurden Temperaturen von 0°C bis +100°C angewandt. Der größte Nachteil bei diesen Verfahren sind Ausbeuteverluste durch Oligomerisierung des Olefins. Beschrieben wird insbesondere ein Verfahren zur Herstellung von tert-Dodecylmercaptan aus **Tetrapropen** und Schwefelwasserstoff bei -40°C in Gegenwart von Bortrifluorid oder Aluminiumtrichlorid als Katalysator.

Für die Herstellung eines Mercaptangemischs durch Schwefelwasserstoff-Addition unter Einsatz eines **Triisobuten-Feedstocks (TIB)** werden in der Literatur verschiedenste Reaktionsbedingungen genannt.

In US-A-2,531,602 wird die Addition von Verbindungen des Typs H-SR, wobei R für Wasserstoff oder einen organischen Rest steht, an verschiedene Olefine und Olefin-Feedstocks beschrieben. Zum Reaktionsmechanismus wird ausgeführt, dass die Addition bei Abwesenheit von Peroxiden und anderen radikalbildenden Substanzen nach Markovnikov verläuft und der Rest -SR an das Kohlenstoffatom mit den meisten Alkylsubstituenten gebunden wird. Auf diese Weise erhält man aus linearen Olefinen sekundäre Mercaptane bzw. sekundäre Thioether und aus verzweigten Olefinen tertiäre Mercaptane bzw. tertiäre Thioether. Da die ungesättigten Edukte jedoch häufig Peroxide enthalten, wird beschrieben, dass auch eine Anlagerung von Schwefelwasserstoff gegenläufig zur Markovnikov-Regel zu erwarten sei. US-A-2,531,602 beschreibt nun, dass die entsprechend der Markovnikov-Regel erwarteten Mercaptane und Thioether auch erhalten werden können, ohne dass die Peroxide aus den Olefinen entfernt zu werden brauchen: Hierzu wird die Umsetzung bei milden Temperaturen und in Gegenwart von wasserfreien Katalysatoren, bevorzugt wasserfreiem Aluminiumchlorid durchgeführt. Die erhaltenen Ausbeuten sind jedoch nicht zufriedenstellend: In Beispiel III wird bei Einsatz von 1,57 Mol % (entsprechend 1,25 Gew. %) AlCl₃ bezogen auf TIB nach einer Reaktionszeit von 0,25 h bei einer Durchschnittstemperatur von -23°C tert.-Dodecylmercaptan nur in einer Ausbeute von 50% erhalten. Aufgrund der sauren Katalyse ist davon auszugehen, dass es bei der Addition auch zu Umlagerungen der Doppelbindung und des Kohlenstoffgerüsts kommen kann, so dass die Addition des Restes -SR auch an einer anderen Stelle des Moleküls erfolgen kann als aufgrund der ursprünglichen Lage der Doppelbindung zu erwarten wäre. Da diese Additionsreaktion reversibel ist, kann es auch nach erfolgter Additionsreaktion über eine Reaktionsfolge von Eliminierungs- und Umlagerungsreaktionen zu einer Wanderung der Doppelbindung kommen. Wird Schwefelwasserstoff eingesetzt, so ist aufgrund dessen Bifunktionalität auch die Bildung von Thioethern möglich. Eine analytische Charakterisierung des in Beispiel III der US-A-2,531,602 erhaltenen tert.Dodecylmercaptans erfolgt nicht.

Nach DD 137 307 wird die Additionsreaktion von Schwefelwasserstoff an ein reines tertiäres aliphatisches Olefin oder an Isomerengemische bei 10 bis 50°C beschrieben, wobei als Katalysatoren flüssige Komplexverbindungen des Aluminiumchlorids mit aromatischen Ethern, wie Anisol und/oder Phenetol, oder deren Lösungen, eingesetzt werden. Diese Komplexverbindungen werden jedoch nachteiligerweise unter Verwendung von aromatischen Lösungmitteln wie Toluol oder Benzol eingesetzt Bei Umsetzung von Triisobuten mit Schwefelwasserstoff in Gegenwart des AlCl₃-Anisol- oder AlCl₃-Phenetol-Komplexes als Katalysator (AlCl₃-Einsätze von 0,9 bis 2,14 Gew. % bezogen auf Triisobuten) werden bei Temperaturen von 10°C bis 50°C unter Nachdosierung von Schwefelwasserstoff Ausbeuten von 91 bis 96 % an tert.-Dodecylmercaptan erhalten. Nachteile des Verfahrens sind somit längere Reaktionszeiten von ca. 30 min und die Verwendung von aromatischen Lösungsmitteln wie Toluol oder Benzol oder von Ethern. Es ist zudem nicht erkennbar, welche Isomerenzusammensetzung das entstehende Mercaptangemisch besitzt und ob dieses als Molekulargewichtsregler für die Herstellung von Kautschuken, insbesondere Nitrilkautschuk, geeignet ist.

Gemäß DD 160 222 erfolgt die Schwefelwasserstoffaddition an tertiäre Olefinen in Gegenwart einer flüssigen Komplexverbindung des AlCl₃ mit Alkylaromaten und Salzsäure, vorzugweise Toluol und/oder Ethylbenzol und/oder Diethylbenzol. In den Beispielen wird **Triisobuten** mit Schwefelwasserstoff im Temperaturbereich von 10-50°C umgesetzt, wobei als Katalysator eine Komplexsalzlösung aus AlCl₃/Toluol /HCl (Molverhältnis: 1 : 3 : 0,6) verwendet wird. Bei Einsatz von 0,31 bis 0,83 Gew. % AlCl₃ bezogen auf Triisobuten werden nach 30 Minuten Ausbeuten an tert.-Dodecylmercaptan im Bereich von 91 bis 93 % erhalten. Die Nachteile des Verfahrens bestehen auch hier somit in längeren Reaktionszeiten und der Verwendung aromatischer Lösungmittel wie Toluoyl. Des Weiteren wird auch in der DD 160 222 keinerlei Aussage zu der Isomerenverteilung im tert.-Dodecylmercaptangemisch gemacht. Auch eine potentielle Anwendung als Molekulargewichtsregler findet keine Erwähnung.

Nach US-A-3,137,735 wird die Addition von Schwefelwasserstoff an **Triisobuten** unter Verwendung eines Komplexes von BF₃ mit H₃PO₄ und einem Alkohol, vorzugsweise Methanol, bei Temperaturen von -90°F bis 0°F in einem kontinuierlichen Verfahren durchgeführt. Entscheidender Nachteil des Verfahrens ist ein sehr niedriger TIB-Umsatz im Bereich von 9 bis maximal 25 mol % pro Reaktionszyklus. Es ist nicht erkennbar, welche Zusammensetzung das entstehende Mercaptangemisch besitzt.

Nach SU 518 489 setzt man als Katalysator Ethylaluminiumdichlorid in Isopentan ein. Nachteile des Verfahrens sind die hohen Mengen an Katalysator und die Verwendung von Lösungsmitteln. Über die Zusammensetzung des tert.-Dodecylmercaptangemischs und seine Einsetzbarkeit als Molekulargewichtsregler wird nichts offenbart.

Nach WO-A-2005/082846 erfolgt die Umsetzung von **trimerisiertem n-Buten** mit Schwefelwasserstoff in kontinuierlicher Fahrweise bei Temperaturen von 10 bis 250°C, vorzugsweise bei 50 bis 150°C, bei einem Druck von 15 bar mit den unterschiedlichsten Katalysatoren, wobei eine Feedstockfraktion mit engem Siedebereich ausgewählt wird. Als Katalysatoren werden bevorzugt polymergebundene Säuren wie z. B. saure Kationenaustauscherharze eingesetzt. Die Wirkung des auf diese Weise hergestellten Mercaptangemischs wird bei der Herstellung von Styrol/Butadien-Copolymeren demonstriert. Nachteil des Verfahrens sind Ausbeuteverluste durch Verwendung eines **trimerisierten n-Buten** Feedstocks mit engem Siedebereich. Das Mercaptangemisch wird strukturell in keiner Weise definiert, sondern ausschließlich über das Siedetemperatur-Verhalten.

Gemäß GB 823,824 erfolgt die Schwefelwasserstoffaddition an verschiedene Olefin-Feedstocks in Gegenwart eines BF₃-Methanol Addukts als Katalysator bei 0 bis 5°C in diskontinuierlicher Fahrweise, wobei Schwefelwasserstoff nachdosiert wird. Als Olefin-Feedstock sind Tetrapropen und Triisobuten geeignet. Bei Verwendung von Triisobuten als Olefin-Feedstock resultiert eine Rohmercaptanmischung mit einem Gehalt an tert.-Dodecylmercaptan von 38 %. Nachteile des Verfahrens sind die niedrige Ausbeute an tert.-Dodecylmercaptan sowie extrem lange Reaktionszeiten von bis zu 20 Stunden. Aussagen zur Isomerenverteilung im erhaltenen tert.-Dodecylmercaptangemisch werden nicht gemacht.

Die Offenbarung der GB 823,823 unterscheidet sich von derjenigen aus GB 823,824 durch die Verwendung eines Katalysators auf Basis eines BF₃-Addukts an Diethylether. Als Olefin-Feedstock sind wiederum Tetrapropen und Triisobuten geeignet. Bei Verwendung von **Triisobuten** als Olefin-Feedstock wird nach der Lehre von GB 823,823 eine Rohmercaptanmischung mit einem Gehalt an tert.-Dodecylmercaptan von 49 % erhalten. Für eine großtechnische Anwendung des Verfahrens ist die Ausbeute an tert.-Dodecylmercaptan somit zu niedrig. Auch die Reaktionszeiten sind mit bis zu 20 Stunden erneut nicht akzeptabel.

In JP 07-316126**,** JP 07-316127**,** JP 07-316128 werden weitere Varianten zur Addition von Schwefelwasserstoff an einen **Triisobuten**-Feedstock beschrieben, der aus 2-(2,2-Dimethylpropan)-4-4Dimethylpenten-1 und/oder 2,2,4,6,6-Pentamethylhepten-3 besteht. Alle Verfahren haben das gemeinsame Ziel, das spezielle tert.Dodecylmercaptan-Isomer 2,2,4,6,6-Pentamethylheptan-4-thiol in hoher Reinheit herzustellen.

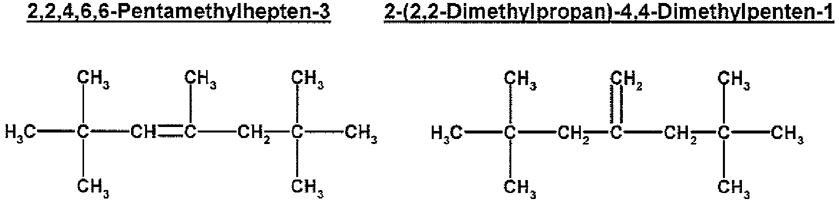

Gemäß JP 07-316126 erfolgt die Umsetzung des zuvor genannten Triisobuten-Feedstocks, in dem die beiden genannten Isomeren in beliebigem Verhältnis zueinander vorliegen können, mit Schwefelwasserstoff unter Druck in Gegenwart von Säuren als Katalysator, wobei die Umsetzung durch ein Abstoppreagenz wie Natriumcarbonat beendet wird, bevor das Reaktionsgemisch auf Normaldruck gebracht wird. Als Säuren können Protonensäuren oder Lewis-Säuren, bevorzugt BF₃ oder ein Komplex auf Basis von BF₃, eingesetzt werden. In den Beispielen wird bei einem Reaktionsdruck im Bereich von 5 bis 10 bar ausgehend von Triisobuten der zuvor genannten Zusammensetzung und einem BF₃ Addukt eine Ausbeute von 54 bis 62 % 2,2,4,6,6-Pentamethylheptan-4-thiol erhalten, wobei jeweils 35 bis 40 % Triisobuten nicht umgesetzt bleiben. Substanzieller Nachteil sind lange Reaktionszeiten im Bereich von 2 bis 6 Stunden.

Gemäß JP 07-316127 erfolgt die Addition von Schwefelwasserstoff in Gegenwart eines Carbonsäure-Addukts an BF₃. Der Triisobuten-Feedstock besteht in diesem Fall zu 50-100 % aus 2-(2,2-Dimethylpropan)-4,4-Dimethylpenten-1 und gegebenenfalls bis zu 50% aus 2,2,4,6,6-Pentamethylhepten-3. Dieses Verhältnis der beiden Isomere zueinander wird als essentiell belegt, um in Gegenwart des Carbonsäure-Addukts von BF₃ 2,2,4,6,6-Pentamethylheptan-4-thiol in Ausbeuten von 56 bis 61 % zu erhalten. Wiederum bleiben 33 bis 40 % des Triisobuten-Feedstocks nicht umgesetzt. Auch die Reaktionszeiten dieses Verfahrens sind deutlich zu lang und liegen bei 2 bis 6 Stunden.

Gemäß JP 07-316128 erfolgt die Addition von Schwefelwasserstoff an einen mit Lewis-Basen verunreinigten Triisobuten-Feedstock in Gegenwart einer Protonensäure und einer Lewis Säure. In dem speziellen mit Lewis-Basen verunreinigten Triisobuten-Feedstock können die beiden zuvor genannten Isomere in beliebigem Verhältnis zueinander vorliegen. Er wird durch Rückgewinnung aus einem ersten Schwefelwasserstoff-Additionszyklus erhalten, indem ein zunächst Lewis-Basen freier Triisobuten-Feedstock mit Schwefelwasserstoff in Gegenwart einer Addukts aus einer LewisSäure wie BF₃ und einer Lewis Base wie z.B. n-Butylether umgesetzt wird. Bei Einsatz des speziellen mit Lewis-Basen verunreinigten Triisobuten-Feedstocks wird 2,2,4,6,6-Pentamethylheptan-4-thiol in einer Ausbeute von 32 % erhalten. 62 % des Triisobutens bleiben nicht umgesetzt zurück. Die Reaktionszeiten sind für eine großtechnische Umsetzung erneut zu lang und liegen bei 6 Stunden.

Bei Verwendung eines C₁₂-Feedstocks auf Basis von **tetramerem Propen (Tetrapropylen oder Tetrapropen)** werden für die Schwefelwasserstoffaddition unterschiedliche Reaktionsbedingungen angewandt, wie aus der nachfolgend zitierten wissenschaftlichen Literatur sowie Patentliteratur hervorgeht.

In Chemical Engineering Progress, Vol 59, no. 7, 1963, 68-73 werden Ergebnisse systematischer Arbeiten zur Addition von Schwefelwasserstoff an einen Tetrapropen-Feedstock untersucht. Die Durchführung des Verfahrens erfolgt bei 120°C bis 160°F (49 bis 71°C) in Gegenwart von gasförmigem Bortrifluorid als Katalysator. Ob und ggf. welchen Einfluss der Wassergehalt auf das Ergebnis der Umsetzung hat, wird nicht beschreiben. Beschrieben wird, dass die Qualität unterschiedlicher Tetrapropen-Feedstocks einen starken Einfluss auf die Ausbeuten hat.

Nach FR 2,094,239 setzt man einen Tetrapropen-Feedstock mit Schwefelwasserstoff bei Normaldruck und Temperaturen von -5 bis +5°C in einem kontinuierlichen Prozess um. Bei der Umsetzung des Schwefelwasserstoffs fallt in der Hochsiederfraktion als Nebenprodukt ein Thioether an, in dem das als Katalysator verwendete Aluminiumtrichlorid gelöst wird. Die Tetrapropeniimsätze pro Reaktionszyklus liegen bei ca. 90%. Nachteil des Verfahrens sind hohe Katalysatormengen (170 g AlCl₃ pro 10 1 Tetrapropen entsprechend ca. 1,12 Mol %) und Verweilzeiten von 2 Stunden.

Nach EP-A-0 329 521 erfolgt die Mercaptanaddition an Tetrapropylen oder Triisobutylen kontinuierlich im Temperaturbereich von 0°C bis 35°C bei einem Druck von 1 bis 10 bar unter Verwendung polymerfixierter Sulfonsäuren, wobei getrocknete Kationenaustauscherharze sowie perfluorsulfonsäurehaltige Copolymere von Tetrafluorethylen bevorzugt sind. Bei Verwendung eines Triisobuten-Feedstocks werden bei 10 bar im Temperaturbereich von 10 bis 45°C in einem Reaktionszyklus nur 50,7 % bis 43,6% des Triisobutens umgesetzt. Zu den Katalysatorstandzeiten wird keine Aussage gemacht.

In US-A-2,951,875 wird die Addition von Schwefelwasserstoff an Propylen-Homopolymere oder Oligomere beschrieben. Eine Depolymerisation von Propylenoligomeren kann dadurch vermieden werden, dass bei Temperaturen im Bereich 25 bis 100°C mit einem aktivierten Al₂O₃/SiO₂-Katalysators gearbeitet wird.

Gemäß US-A-4,102,931 können tert.-Mercaptan-Gemische durch Umsetzung eines Tetrapropen-Feedstocks mit Schwefelwasserstoff in Gegenwart synthetischer Zeolithe hergestellt werden. Die Standzeit dieses Katalysators ist abhängig von der Restfeuchte der verwendeten Rohstoffe. Der Schwefelwasserstoff wird kontinuierlich mit einem Tetrapropen-Feedstock im Molverhältnis 10/1 bei 85 bis 95°C und 9 bar mit Hilfe eines synthetisch hergestellten Zeoliths umgesetzt. Bei Verwendung von getrocknetem Schwefelwasserstoff und trockenem Tetrapropen liegen die Olefinumsätze bei einem Durchgang bei > 90%. In der Praxis ist die Standzeit des speziellen Katalysators stark abhängig von der Restfeuchte der verwendeten Rohstoffe. Dringt Feuchtigkeit ein, so muss die Anlage abgestellt werden, um den Katalysator durch Hitzebehandlung bei 240°C zu regenerieren, wobei die Regenerierungstemperatur des Katalysators signifikant niedriger liegt als bei als Stand der Technik dargestellten Aluminiumsilikaten (500°C) ist. In US-A-4,102,931 werden keine Angaben zu den Verweilzeiten gemacht, bei denen die hohen Olefinumsätze erzielt werden. Auch ist nicht geklärt, ob die Ergebnisse auf Triisobuten übertragbar sind.

Gemäß EP-A-0 101 356 wird die Schwefelwasserstoffaddition an Tetrapropen bei einer Temperatur von 45 bis 75°C und einem Druck von 10 bis 16 bar kontinuierlich durchgeführt. Der Wassergehalt des Kationenaustauscherharzes muss kleiner 0,5 Gew. % sein, was durch 6-stündige Trocknung bei 80°C erreicht wird. Bei Verwendung eines Harzes mit 1,6 Gew. % Wasser wird der Tetrapropenumsatz von 90% auf 80% reduziert. Zur Isomerenverteilung im erhaltenen tert.-Dodecylmercaptan-Gemisch finden sich keinerlei Angaben. Ungeklärt ist auch, ob die Ausbeuten auf einen Triisobuten-Feedstock übertragbar wären. Auch die Eignung eines solchen tert.-Dodecylmercaptan-Gemischs als Regler bei der Kautschukherstellung ist nicht erwähnt.

In der WO-A-2005/030710 wird die katalytische Herstellung von tert-Dodecylmercaptan durch Addition von Schwefelwasserstoff an ein spezielles Olefin-Gemisch auf Basis eines **Hexen-Dimeren** beschrieben. Dieses durch Nickel-katalysierte Hexendimerisierung erhaltene Olefin-Gemisch umfasst mindestens 10-18 Gew.% von n-Dodecan abgeleitetes Olefin, 25-40 Gew.% von 5-Methyl-n-undecan abgeleitetes Olefin, 25-40Gew.% von 4-Ethyl-n-decan abgeleitetes Olefin, 2-78 Gew.% von 5,6-Di-methyl-n-decan abgeleitetes Olefin, 5-12 Gew.% von 5-Ethyl-6-methyl-n-nonan abgeleitetes Olefin, 1-5- Gew.% von 4,5-Di-ethyl-n-octan abgeleitetes Olefin und höchstens 5 Gew.% anderer Kohlenwasserstoffe. Als Hintergrund für diese spezielle Synthese wird angegeben, dass Polymerdispersionen auf der Basis des bekannten tert.-Dodecylmercaptans häufig einen unangenehmen Geruch aufwiesen und dass bei tert-Dodecylmercaptan, welches aus trimerem Isobuten hergestellt wird, der Geruch im Allgemeinen noch stärker sei als bei Verwendung von tert-Dodecylmercaptan, das aus tetramerem Propen hergestellt wird. Das gemäß WO-A-2005/030710 erhaltene Mercaptangemisch soll nun die Reduktion des Eigengeruchs von Emulsionsspolymerisaten auf der Basis von Butadien, Styrol und Acrylnitril ermöglichen. Die Polymerisation erfolgt bei 85 bis 95°C. Aus der WO-A-2005/030710 ist nicht zu entnehmen, ob sich das besondere Mercaptangemisch zur Molekulargewichtseinstellung bei der Kaltpolymerisation von NBR eignet.

In den zuvor genannten Literaturstellen wird die Addition von Schwefelwasserstoff an unterschiedliche C₁₂-Olefin-Feedstocks und unter verschiedensten Verfahrensbedingungen beschrieben. Eine Vielzahl von weiteren möglichen Einflussgrößen wird aufgeführt, so z.B. Art und Zusammensetzung der Verunreinigungen, Art und Menge des Katalysators und von Zusätzen bzw. Cokatalysatoren, Art und Menge an Lösungsmitteln, Reaktionsbedingungen (Temperatur, Druck und Reaktionszeit), Art des Verfahrens sowie die Art zur Abstoppung und Beendigung der Reaktion. Unklar bleibt in vielen Fällen die Zusammensetzung des eingesetzten Olefin-Gemischs/Feedstocks und Begrifflichkeiten werden häufig unscharf benutzt. Nicht untersucht wird auch die Zusammensetzung der erhaltenen Mercaptangemische und entsprechend auch die Frage eines Zusammenhangs zwischen den verschiedenen Einflußgrößen und der Zusammensetzung der erhaltenen Mercaptangemische. Weiterhin ungeklärt bleibt in den meisten Fällen die Frage der Einsetzbarkeit dieser Mercaptane als Molekulargewichtsregler.

Die Aufgabe der vorliegenden Erfindung bestand darin, ein verbessertes Verfahren bereitzustellen, durch das ein Mercaptangemisch zugänglich wird, welches auf Basis kommerziell verfügbarer Olefin-Gemische herstellbar ist und das bereits bei Einsatz geringer Mengen eine exzellente Regelung des Molekulargewichts im Rahmen der Herstellung von Kautschuken, insbesondere von Nitrilkautschuken erlaubt.

Überraschenderweise gelingt es, durch ein besonderes Herstellungsverfahren ein neues Mercaptangemisch spezieller Zusammensetzung bereitzustellen, das die genannten Anforderungen erfüllt.

**Gegenstand der vorliegenden Erfindung** ist somit ein Verfahren zur Herstellung eines Gemischs enthaltend
- 2,2,4,6,6-Pentamethylheptanthiol-4,
- 2,4,4,6,6-Pentamethylheptanthiol-2,
- 2,3,4,6,6-Pentamethylheptanthiol-2 und
- 2,3,4,6,6-Pentamethylheptanthiol-3,
   umfassend die Umsetzung von Schwefelwasserstoff mit Triisobuten in einem kontinuierlichen Verfahren bei Temperaturen von 0°C bis -60°C, worin
   (a) der Schwefelwasserstoff vor der Umsetzung einer Trocknung unterzogen wird,
   (b) das eingesetzte Triisobuten einen Wassergehalt von maximal 70 ppm besitzt,
   (c) als Katalysator Bortrifluorid in Mengen von 0,25 bis 1,2 Gew.%, bezogen auf das eingesetzte Triisobuten verwendet wird,
   (d) die Umsetzung in Abwesenheit von mit Bortrifluorid komplexbildenden Verbindungen erfolgt und
   (e) das Reaktionsgemisch im Anschluss an die Umsetzung mit einer wässrigen alkalischen Lösung enthaltend Ammoniak, Amine, Hydroxide, Hydrogencarbonate bzw. Carbonate in gelöster Form in Kontakt gebracht und der Katalysator entfernt wird,
      wobei das
      45 bis 55 Gew.% 2,2,4,6,6-Pentamethylhepten-3,
      30 bis 40 Gew.% 2-(2,2-Dimethylpropyl)-4,4-dimethylpenten-1,
      1 bis 5 Gew.% 2,4,4,6,6-Pentamethylhepten-2,
      1 bis 5 Gew.% 2,4,4,6,6-Pentamethylhepten-1 und
      5 bis 15 Gew.% weitere Verbindungen
      enthält, wobei sich alle vorgenannten Komponenten zu 100 Gew.% aufsummieren.

**Gegenstand der vorliegenden Erfindung ist** ferner ein Gemisch enthaltend 70-85 Flächen % 2,2,4,6,6-Pentamethylheptanthiol-4, 3-6 Flächen% 2,4,4,6,6-Pentamethylheptanthiol-2, 2-5 Flächen % 2,3,4,6,6-Pentamethyllieptanthiol-2, 3-13 Flächen% 2,3,4,6,6-Pentamethylheptanthiol-3 und weiteren Verbindungen, wobei die Summe aller vorgenannten Isomeren und der weiteren Verbindungen 100 Flächen % ergibt.

**Gegenstand der Erfindung** ist ferner die Verwendung des erfindungsgemäßen Gemischs zur Regelung des Molekulargewichts bei Polymerisationen zur Herstellung von Kautschuken, insbesondere zur Herstellung von Nitrilkautschuken.

**Gegenstand der Erfindung** ist ebenso ein Kautschuk, insbesondere ein Nitrilkautschuk, der erhältlich ist durch Polymerisation in Gegenwart des erfindungsgemäßen Gemischs und der 2,2,4,6,6-Pentamethylheptan-4-thio- und/oder 2,4,4,6,6-Pentamethylheptan-2-thio- und/oder 2,3,4,6,6-Pentamethylheptan-2-thio und/oder 2,3,4,6,6-Pentamethylheptan-3-thio-Endgruppen enthält.

### Herstellung des erfindungsgemäßen Mercapatangemischs

Ungeachtet der langen Historie und der vielfältigen Herstellungswege für Gemische von tert.-Dodecylmercaptanen wurde durch die vorliegende Erfindung erstmals die kritischen Einflussfaktoren gefunden, deren gleichzeitige Einhaltung eine klare Steuerung der Zusammensetzung und Qualität des erfindungsgemäßen Gemischs ermöglicht.

Hierzu gehört, dass das eingesetzte einen Wassergehalt von maximal 70 ppm aufweist. Bevorzugt beträgt dieser Wassergehalt maximal 50 ppm, besonders bevorzugt liegt er bei weniger als 50 ppm und ganz besonders bevorzugt bei maximal 40 ppm. Sofern der Triisobuten-Feedstock diesen Wassergehalt nicht bereits besitzt, wird er vor der Umsetzung mit Schwefelwasserstoff eingestellt. Diese Einstellung des Wassergehalts ist beispielsweise über Destillation möglich oder indem das mit Molekularsieben oder Zeolithen in Kontakt gebracht wird. Die Bestimmung des Wassergehalts kann durch die dem Fachmann bekannte Karl-Fischer-Titration erfolgen.

Das zur Umsetzung mit Schwefelwasserstoff eingesetzte **Triisobuten** (**"TIB"**) enthält die vier Isomeren
1) 2,2,4,6,6-Pentamethylhepten-3,
2) 2-(2,2-Dimethylpropyl)-4,4-dimethylpenten-1,
3) 2,4,4,6,6-Pentamethylhepten-2 und
4) 2,4,4,6,6-Pentamethylhepten-1.

Diese nachfolgend mit ihren Strukturformeln dargestellten Isomeren können gemäß bekannten analytischen Methoden, so z.B. über NMR oder durch gaschromatographische Trennung in Kombination mit Massenspektroskopie, identifiziert werden.

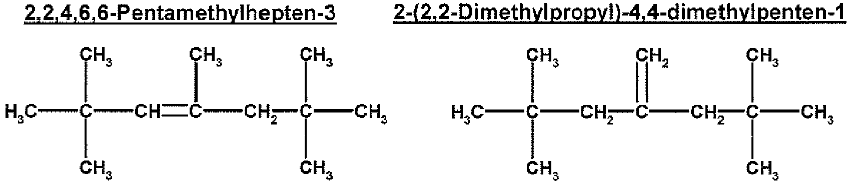

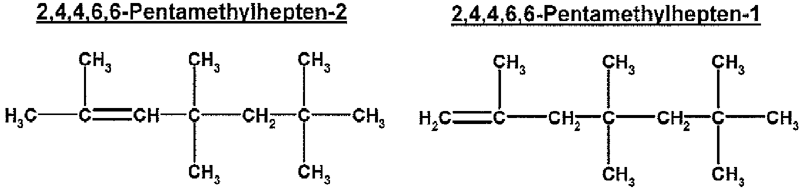

Neben diesen vier Isomeren enthält das eingesetzte Triisobuten üblicherweise gewisse Mengen weiterer Bestandteile.

Die Herstellung des Triisobutens kann ausgehend von dem Fachmann bekannten Methoden durch Steuerung der Verfahrensparameter erfolgen, wie z.B. in der DE 1 199 761 B beschrieben, wobei als Katalysator für die Oligomerisierung beispielsweise ein Kationenaustauscher in protonierter Form eingesetzt wird. Für den speziellen Wassergehalt des Triisobutens ist, wie oben beschrieben, separat Sorge zu tragen.

Der für die erfindungsgemäße Umsetzung verwendete **Schwefelwasserstoff** wird bevorzugt durch Umsetzung von kommerziell erhältlichem Natriumhydrogensulfid mit Schwefelsäure in-situ hergestellt. Schwefelsäure, die bei der Herstellung mitgerissen wird, kann beispielsweise durch eine Wasserwäsche entfernt werden.

Für die Zusammensetzung und Qualität des erfindungsgemäßen Gemischs ist es ferner wichtig, dass der eingesetzte Schwefelwasserstoff vor der erfindungsgemäßen Umsetzung einer Trocknung unterzogen wird. Hierdurch wird dem Schwefelwasserstoff das Wasser entzogen. Die Trocknung erfolgt beispielsweise durch kryostatische Entfernung des im Schwefelwasserstoff enthaltenen Wassers. Hierfür wird beispielsweise in einer ersten Stufe das im Schwefelwasserstoff enthaltene Wasser durch Abkühlung auf Temperaturen im Bereich von -10°C bis 30°C, bevorzugt im Bereich von 0°C bis 20°C, besonders bevorzugt im Bereich von 3 °C bis 18 °C und insbesondere im Bereich von 10°C bis 15°C auskondensiert. In der zweiten Stufe wird der Schwefelwasserstoff erneut auf eine Temperatur im Bereich von -15°C bis -50°C, bevorzugt im Bereich von -10GC bis -25 °C abgekühlt. Hierdurch wird das restliche im Schwefelwasserstoff enthaltene Wasser ausgefroren. Die Effizienz der Wasserentfernung wirkt sich direkt auf den Einsatz von BF₃ bei der Schwefelwasserstoffaddition aus, d.h. hierdurch wird eine hohe Katalysatoreffizienz erzielt. Der Gegenstand der Erfindung würde nicht verlassen, wenn in das erfindungsgemäße Verfahren Schwefelwasserstoff eingesetzt würde, der bereits in entsprechend trockener Form, d.h. in vom Wasser befreiter Form, käuflich bezogen würde.

Vor der Zugabe des Katalysators wird Schwefelwasserstoff in flüssiger Form mit dem Triisobuten gemischt und gekühlt. Das Molverhältnis von Schwefelwasserstoff zu Triisobuten liegt im erfindungsgemäßen Verfahren im Bereich von (1,1-10,0) : 1, bevorzugt im Bereich von (1,1- 5,0) : 1, besonders bevorzugt im Bereich (1,1-3,0) : 1 und insbesondere im Bereich von (1,2-2,8) : 1.

Die Temperatur der Mischung von Schwefelwasserstoff und Triisobuten liegt vor der Zugabe des Katalysators im Bereich von -50°C bis 0°C, bevorzugt im Bereich von - 40°C bis -30°C und besonders bevorzugt im Bereich von -35 bis -25 °C.

Für die Katalyse wird **Bortrifluorid** verwendet. Hierbei hat es sich bewährt, Bortrifluorid mit einem Reinheitsgrad von mindestens 98%, bevorzugt >98%, besonders bevorzugt >99% einzusetzen. Bortrifluorid ist dabei ohne den Zusatz von solchen Verbindungen bzw. Additiven zu verwenden, die mit Bortrifluorid Komplexe bilden. Derartige Additive umfassen Verbindungen, die z.B. auch als Lösungsmittel oder als Cokatalysatoren fungieren. Hierzu zählen beispielsweise Ether in Form von Dialkylethern, wie z.B. Diethylether, oder in Form von Alkylarylethern, wie z.B. Phenetol oder Anisol, oder aromatische Verbindungen, wie z.B. Toluol oder Benzol. Die Dosierung des Bortrifluorids erfolgt gasförmig, üblicherweise bei einem Überdruck im Bereich von 5 bis 10 bar, vorzugsweise im Bereich von 7 bis 8 bar.

Die Menge an Bortrifluorid bezogen auf das eingesetzte Triisobuten beträgt maximal 1,5 Gew.%, bevorzugt 0,25 bis 1,2 Gew.%, besonders bevorzugt 0,5 bis 1,0 Gew. %, insbesondere 0,6 bis 0,9 Gew.% und insbesondere bevorzugt 0,65 bis 0,85 Gew. %.

Das erfindungsgemäße Verfahren wird in einem Temperaturbereich von -10°C bis -60°C durchgeführt.

Die katalytische Addition von Schwefelwasserstoff an Triisobuten erfolgt kontinuierlich. Vorzugsweise wird in einem oder in zwei hintereinander geschalteten Rohrreaktoren gearbeitet. Besonders bevorzugt sind zwei hintereinander geschaltete Rohrreaktoren.

Das Längen/Durchmesser-Verhältnis des ersten Rohrreaktors beträgt üblicherweise (5-20):1, vorzugsweise 10:1. Die Verweilzeiten in diesem Reaktor betragen üblicherweise 0,5 bis 5 min, vorzugsweise 1 bis 3 min. Die Eintrittstemperatur in den 1. Reaktor liegt üblicherweise im Bereich von -25°C bis -35°C und bevorzugt im Bereich von -27,5°C bis -32,5°. Ein Mittelwert von ca. - 30°C hat sich bewährt. Die in diesem ersten Reaktor anfallende Reaktionswärme wird durch externe Kühlung entfernt. Bevorzugt ist Siedekühlung durch verdampfenden Schwefelwasserstoff, der in das Reaktionsgemisch zurückgeführt wird.

Das Längen/Durchmesser-Verhältnis des zweiten Rohrreaktors beträgt üblicherweise (100-200):1, vorzugsweise (120-180):1. Dieser Rohrreaktor hat beispielsweise eine gewendelte Form und ist kühlbar. Die Verweilzeiten in diesem Reaktor liegen üblicherweise im Bereich von 0,5 bis 5 min, vorzugsweise im Bereich von 1 bis 3 min. Die Eintrittstemperatur des Reaktionsgemischs in den zweiten Rohrreaktor liegt im Bereich von -30°C bis 0°C , bevorzugt im Bereich von -25°C bis -5°C und die Austrittstemperatur bei -40°C bis -60°C.

Nach Beendigung der Umsetzung wird das Reaktionsgemisch mit einer wässrigen, alkalischen Lösung in Kontakt gebracht. Es hat sich bewährt, das Reaktionsgemisch in diese wässrige, alkalische Lösung einzuleiten. Die wässrige alkalische Lösung hat eine Temperatur im Bereich von 10°C bis 100°C, bevorzugt im Bereich von 30°C bis 90°C und insbesondere im Bereich von 50°C bis 80°C.

Es handelt sich um eine wässrige Lösung, die Ammoniak, Amine, Hydroxide, Hydrogencarbonate bzw. Carbonate in gelöster Form enthält. Bevorzugte Beispiele sind Ammoniak, Natriumhydroxid, Kaliumhydroxid, Natriumcarbonat, Kaliumcarbonat, Natriumhydogencarbonat und Kaliumhydrogencarbonat. Insbesondere bevorzugt sind Natriumhydroxid und Kaliumhydroxid. Die Konzentration dieser wässrigen alkalischen Lösung beträgt bis zu 20 Gew. %, vorzugsweise 1 bis 10 Gew. %, besonders bevorzugt 3 bis 5 Gew. %. Durch den Kontakt zwischen Reaktionsgemisch und der wässrigen alkalischen Lösung wird die Umsetzung gestoppt und der Katalysator Bortrifluorid extraktiv aus dem Reaktionsgemisch entfernt.

Während des Kontakts zwischen Reaktionsgemisch und wässriger alkalischer Lösung wird die Temperatur im Bereich von 10°C bis 100°C, bevorzugt im Bereich von 30°C bis 90°C und insbesondere im Bereich von 50°C bis 80 °C z. B durch die Einleitung von Wasserdampf gehalten, wodurch eine Entfernung des nicht umgesetzten Schwefelwasserstoffs durch Austreibung möglich wird. Dieser nicht umgesetzte Schwefelwasserstoff kann, sofern gewünscht, in die erfindungsgemäße Umsetzung zurückgeführt werden.

Im Anschluss an den zuvor beschriebenen Abstopp/Extraktions- und Austreib-Schritt kann das erfindungsgemäße Roh-Mercaptangemisch unter Ausnutzung der Dichteunterschiede mit Hilfe eines Separators (Mixer/Settler) kontinuierlich von der wässrigen alkalischen Lösung abgetrennt werden.

Gegebenenfalls wird das Roh-Gemisch mit entionisiertem Wasser ein oder mehrmals gewaschen.

Aus dem Roh-Gemisch können nicht umgesetztes sowie Hochsieder durch kontinuierliche fraktionierende Destillation abgetrennt werden. Diese Destillation wird vorzugsweise unter verringertem Druck in einer mehrstufigen Kolonne durchgeführt.

Nach einem Reaktionszyklus der erfindungsgemäßen Umsetzung beträgt der Umsatz des Triisobutens 75 bis 90 %, vorzugsweise 80 bis 85 %.

Nicht umgesetztes Triisobuten wird abgetrennt, gesammelt und kann in einem 2. Reaktionszyklus noch einmal unter den zuvor beschriebenen Bedingungen umgesetzt werden. Beim 2. Durchgang wird das eingesetzte Triisobuten zu 60 % bis 70 %, vorzugsweise 63 bis 65 % umgesetzt.

Der Triisobuten-Gesamtumsatz aus einem 1. und 2. Reaktionsdurchgang beträgt 85 bis 99 %, vorzugsweise 87 bis 97 %.

In Summe beträgt die Ausbeute an destillativ gereinigtem Gemisch nach 2 Reaktionsdurchgängen 92 ± 5 % bezogen auf das eingesetzte Triisobuten.

Sofern zwei Reaktionsdurchgänge durchgeführt werden, können die erfindungsgemäßen Gemische aus dem 1. und 2. Reaktionsdurchgang vereinigt werden.

Das erfindungsgemäße Gemisch enthält 70-85 Flächen% 2,2,4,6,6-Pentamethyllieptanthiol-4, 3-6 Flächen% 2,4,4,6,6-Pentamethylheptanthiol-2, 2-5 Flächen% 2,3,4,6,6-Pentamethylheptanthiol-2, 3-13 Flächen% 2,3,4,6,6-Pentamethylheptanthiol-3 und weiteren Verbindungen, wobei die Summe aller vorgenannten Isomeren und der weiteren Verbindungen 100 Flächen% ergibt, die nachfolgend formelmäßig dargestellt sind.

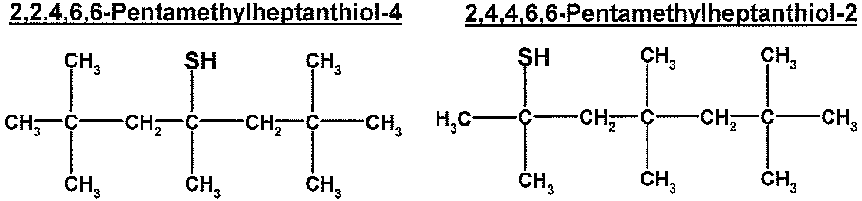

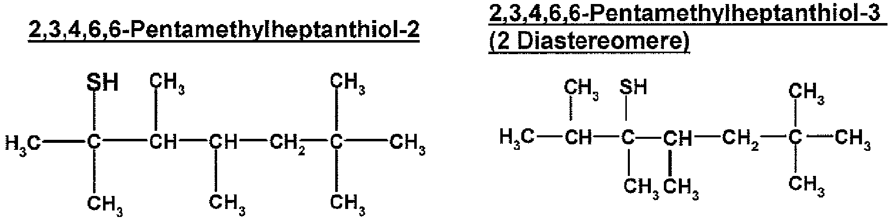

Die Zuordnung der chemischen Strukturformeln zu den einzelnen, mittels Gaschromatographie aufgetrennten Fraktionen, erfolgt mit Hilfe der Massenspektrometrie.

Die Gaschromatographie wird dabei unter FID-Detektion durchgeführt. In der Praxis wird dabei üblicherweise so vorgegangen, dass jeweils 50 µl des erfindungsgemäßen Gemischs mit 5 mL Toluol verdünnt werden und von dieser Lösung 0,2 µL gaschromatographisch untersucht werden. Für die Durchführung der Analysen werden folgende Bedingungen angewandt:

| | |
|---|---|
| Gaschromatograph: | Fison Trace GC |
| Trennsäule: | Stabilwax-DB (Restek) 30m, 0,25mm ID, 0,25µm df |
| Detektortemperatur: | 260°C |
| Injektortemperatur: | 170°C |

| | |
|---|---|
| Ofentemperatur: | 60°C, 2min isotherm Aufheizrate: 5°C/min 190°C Endtemperatur, 1min isotherm |
| Trägergas: | Helium, 1,0 mL Säulenfluss |
| Split: | 50 mL/min |

Anschließend wurde die chemische Struktur der mittels Gaschromatographie aufgetrennten Fraktionen des erfindungsgemäßen Isomerengemischs durch Massenspektroskopie bestimmt.

Basierend auf dieser Analyse besteht das erfindungsgemäße Gemisch bevorzugt aus: 70 - 85 Flächen%, besonders bevorzugt 75 - 83 Flächen% 2,2,4,6,6-Pentamethylheptanthiol-4, 3 - 6 Flächen%, besonders bevorzugt 4 - 5,5 Flächen% 2,4,4,6,6-Pentamethylheptanthiol-2, 2 - 5 Flächen%, besonders bevorzugt 3 - 4,5 Flächen% 2,3,4,6,6-Pentamethylheptanthiol-2, 3 - 13 Flächen%, besonders bevorzugt 3,5 - 12 Flächen% 2,3,4,6,6-Pentamethylheptanthiol-3 sowie weiteren Verbindungen, wobei die Summe aller Isomeren und der weiteren Verbindungen 100 ergibt.

Das erfindungsgemäße Gemisch enthaltend 2,2,4,6,6-Pentamethylheptanthiol-4, 2,4,4,6,6-Pentamethylheptanthiol-2, 2,3,4,6,6-Pentamethylheptanthiol-2 und 2,3,4,6,6-Pentamethyl-heptanthiol-3 ist bevorzugt erhältlich durch Umsetzung von Schwefelwasserstoff mit Triisobuten in einem kontinuierlichen Verfahren bei Temperaturen im Bereich von 0°C bis -60°C, worin
(a) der Schwefelwasserstoff vor der Umsetzung einer Trocknung unterzogen wird,
(b) das eingesetzte Triisobuten einen Wassergehalt von maximal 70 ppm besitzt,
(c) als Katalysator Bortrifluorid in Mengen von maximal 0,25 bis 1,2 Gew.%, bezogen auf das eingesetzte Triisobuten verwendet wird,
(d) die Umsetzung in Abwesenheit von mit Bortrifluorid komplexbildenden Verbindungen erfolgt und
(e) das Reaktionsgemisch im Anschluss an die Umsetzung mit einer wässrigen alkalischen Lösung enthaltend Ammoniak, Amine, Hydroxide, Hydrogencarbonate bzw. Carbonate in gelöster Form in Kontakt gebracht und der Katalysator entfernt wird,
   wobei das Triisobuten
   45 bis 55 Gew.% 2,2,4,6,6-Pentamethylhepten-3,
   30 bis 40 Gew.% 2-(2,2-Dimethylpropyl)-4,4-dimethylpenten-1,
   1 bis 5 Gew.% 2,4,4,6,6-Pentamethylhepten-2,
   1 bis 5 Gew.% 2,4,4,6,6-Pentamethylhepten-1 und
   5 bis 15 Gew.% weitere Verbindungen
   enthält, wobei sich alle vorgenannten Komponenten zu 100 Gew.% aufsummieren.

Die Vorteile des erfindungsgemäßen Verfahrens sind vielfältig:
- Es ist auf Basis eines gut zugänglichen Triisobutens durchführbar und ergibt ein Gemisch, welches die zuvor genannten vier speziellen Isomere beinhaltet.
- Die Herstellung erfolgt auf sehr wirtschaftliche Weise in einem kontinuierlichen Verfahren bei extrem kurzen Verweilzeiten und gleichzeitig sehr niedrigen Katalysatoreinsätzen von maximal 1,5 Gew.%, bezogen auf das eingesetzte Triisobuten. Hierbei wird erfreulicherweise in Abwesenheit von solchen Additiven gearbeitet, die mit dem als Katalysator eingesetzten Bortrifluorid Komplexe bilden und in den Verfahren des Standes der Technik weitere organische, häufig aromatische und damit unerwünschte Bestandteile darstellen.
- Das erfindungsgemäße Gemisch wird in hohen Ausbeuten erhalten.

Das erfindungsgemäße Gemisch lässt sich ferner exzellent zur Regelung des Molekulargewichts bei der Herstellung synthetischer Kautschuke, insbesondere zur Herstellung von Nitrilkautschuken verwenden. Zur Einstellung der gewünschten Molekulargewichte sind dabei nur sehr geringe Mengen nötig.

Das erfindungsgemäße Gemisch eignet sich zur Molekulargewichtseinstellung im Rahmen der Herstellung von Kautschuken auf Basis von Diolefinen wie z.B. Butadien und gegebenenfalls weiteren copolymerisierbaren Monomeren wie z.B. Styrol, α-Methylstyrol, Vinylnaphthalin, Acrylnitril, Methacrylnitril, Methylmethacrylat, Ethylfumarat oder Methylvinylketon, inbesondere durch Emulsionspolymerisation.

Bevorzugt wird das erfindungsgemäße Gemisch zur Molekulargewichtseinstellung im Rahmen der Herstellung von Nitrilkautschuken eingesetzt. Als Monomere werden dabei mindestens ein α,β-ungesättigtes Nitril, mindestens ein konjugiertes Dien und gegebenenfalls ein oder mehrere weitere copolymerisierbare Monomere eingesetzt.

Das konjugierte Dien kann von jeder Natur sein. Bevorzugt werden (C₄-C₆) konjugierte Diene eingesetzt. Besonders bevorzugt sind 1,3-Butadien, Isopren, 2,3-Dimethylbutadien, Piperylen, 1,3-Pentadien oder beliebige Mischungen daraus. Insbesondere bevorzugt sind 1,3-Butadien und Isopren oder Gemische daraus. Ganz besonders bevorzugt ist 1,3-Butadien.

Als α,β-ungesättigtes Nitril kann jedes bekannte α,β-ungesättigte Nitril eingesetzt werden, bevorzugt sind (C₃-C₅)-α,β-ungesättigte Nitrile wie Acrylnitril, Methacrylnitril, 1-Chloracrylnitril, Ethacrylnitril oder Mischungen davon. Besonders bevorzugt ist Acrylnitril.

Einen besonders bevorzugt mit dem erfindungsgemäßen Mercaptangemisch herstellbaren Nitrilkautschuk stellt somit ein Copolymer aus Acrylnitril und 1,3-Butadien dar.

Neben dem konjugierten Dien und dem α,β-ungesättigten Nitril können noch ein oder mehrere weitere dem Fachmann bekannte, copolymerisierbare Monomere eingesetzt werden, z.B. α,β-ungesättigte Mono- oder Dicarbonsäuren, deren Ester oder Amide. Solche Nitrilkautschuke werden üblicherweise auch als carboxylierte Nitrilkautschuke, oder abgekürzt auch als "XNBR" bezeichnet.

Als α,β-ungesättigte Mono- oder Dicarbonsäuren können beispielsweise Fumarsäure, Maleinsäure, Acrylsäure, Methacrylsäure, Crotonsäure und Itakonsäure verwendet werden. Bevorzugt sind dabei Maleinsäure, Acrylsäure, Methacrylsäure und Itakonsäure.

Als Ester der α,β-ungesättigten Carbonsäuren werden beispielsweise Alkylester, Alkoxyalkylester, Hydroxyalkylester oder Mischungen daraus eingesetzt.

Besonders bevorzugte Alkylester der α,β-ungesättigten Carbonsäuren sind Methyl(Meth)acrylat, Ethyl(meth)acrylat, Propyl(meth)acrylat, n-Butyl(Meth)acrylat, t-Butyl(Meth)acrylat, Hexyl(meth)acrylat, 2-Ethlyhexyl(meth)acrylat, Octyl(meth)acrylat und Lauryl(meth)acrylat. Insbesondere wird n-Butylacrylat eingesetzt.

Besonders bevorzugte Alkoxyalkylester der α,β-ungesättigten Carbonsäuren sind Methoxyethyl(meth)acrylat, Ethoxyethyl(meth)acrylat und Methoxyethyl(meth)acrylat. Insbesondere wird Methoxyethylacrylat eingesetzt.

Besonders bevorzugte Hydroxyalkylester der α,β-ungesättigten Carbonsäuren sind Hydroxyethyl(meth)acrylat, Hydroxypropyl-(meth)acrylat und Hydroxy(butyl(meth)acrylat.

Als Ester der α,β-ungesättigten Carbonsäuren werden ferner beispielsweise Polyethylenglykol(meth)acrylat, Polypropylenglykol(meth)acrylat, Glycidyl(meth)acrylat, Epoxy(meth)acrylat und Urethan(meth)acrylat eingesetzt.

Weitere möglich Monomere sind Vinylaromaten wie Styrol, a-Methylstyrol und Vinylpyridin.

Die Anteile an konjugiertem Dien und α,β-ungesättigtem Nitril können für die Herstellung der Nitrilkautschuke in weiten Bereichen variieren. Der Anteil des oder der Summe der konjugierten Diene liegt üblicherweise im Bereich von 20 bis 95 Gew.%, bevorzugt im Bereich von 40 bis 90 Gew.-%, besonders bevorzugt im Bereich von 60 bis 85 Gew. %, bezogen auf das Gesamtpolymer. Der Anteil des oder der Summe der α,β-ungesättigten Nitrile liegt üblicherweise bei 5 bis 80 Gew.%, bevorzugt bei 10 bis 60 Gew.-%, besonders bevorzugt bei 15 bis 40 Gew.-%, bezogen auf das Gesamtpolymer. Die Anteile der Monomere summieren sich jeweils zu 100 Gew.-% auf.

Die zusätzlichen Monomere können in Mengen von 0 bis 40 Gew.% eingesetzt werden. Möglich sind Mengen von 0,1 bis 40 Gew.-%, bevorzugt 1 bis 30 Gew.-%, bezogen auf die Summe der Monomere. In diesem Fall werden entsprechende Anteile des oder der konjugierten Diene und/oder des oder der α,β-ungesättigten Nitrile durch die Anteile dieser zusätzlichen Monomere ersetzt, wobei sich die Anteile aller Monomere weiterhin jeweils zu 100 Gew.-% aufsummieren.

Werden als zusätzliche Monomere Ester der (Meth)acrylsäure eingesetzt, so erfolgt dies üblicherweise in Mengen von 1 bis 25 Gew. %.

Werden als zusätzliche Monomere α,β-ungesättigte Mono- oder Dicarbonsäuren eingesetzt, so erfolgt dies üblicherweise in Mengen von weniger als 10 Gew. %.

Das Verfahren zur Herstellung der Kautschuke kann nach dem Fachmann bekannten Methoden erfolgen. Nitrilkautschuke werden üblicherweise über Emulsionspolymerisation hergestellt.

Der Stickstoffgehalt wird in den so erhaltenen Nitrilkautschuken gemäß DIN 53 625 nach Kjeldahl bestimmt. Aufgrund des Gehalts an polaren Comonomeren sind die Nitrilkautschuke üblicherweise in Methylethylketon bei 20°C ≥ 85 Gew. % löslich.

Die so herstellbaren Nitrilkautschuke weisen Mooney-Viskositäten (ML 1+4 @100°C) im Bereich von 10 bis 150, vorzugsweise von 20 bis 100 Mooneyeinheiten auf. Die Mooney-Viskosität (ML 1+4@100°C) wird mittels eines Scherscheibenviskosimeters nach DIN 53523/3 bzw. ASTM D 1646 bei 100°C bestimmt.

Die Glastemperaturen der Nitrilkautschuke liegen im Bereich von -70°C bis +10°C, vorzugsweise im Bereich von -60°C bis 0°C.

Das erfindungsgemäße Gemisch findet sich aufgrund seiner Funktion als Molekulargewichtsregler in einem gewissen Maß in Form von Endgruppen in den bei der Polymerisation hergestellten Kautschuken, insbesondere den Nitrilkautschuken, wieder. D.h. der Nitrilkautschuk weist 2,2,4,6,6-Pentamethylheptan-4-thio- und/oder 2,4,4,6,6-Pentamethylheptan-2-thio- und/oder 2,3,4,6,6-Pentamethylheptan-2-thio und/oder 2,3,4,6,6-Pentamethylheptan-3-thio-Endgruppen auf.

### BEISPIELE:

### EINGESETZTE MATERIALIEN:

**Triisobuten** (TIB) ist ein trimerisiertes iso-Buten und wird von INEOS, Deutschland bezogen. TIB hat einen Siedebereich von 174 - 189°C; einen Brechungsindex von n_{D}²⁰ = 1,4367, einen Schwefelgehalt < 5 ppm und einen Peroxidgehalt, bestimmt als H₂O₂, < 5 ppm. Der Wassergehalt beträgt 37 ppm.

### Es besteht aus:

| | |
|---|---|
| 48 Gew.% | 2,2,4,6,6-Penlamethylhepten-3, |
| 33 Gew.% | 2-(2,2-Dimethylpropan)-4,4-Dimethylpenten-1, |
| 3 Gew.% | 2,4,4,6,6-Pentamethylhepten-2 und |
| 2 Gew.% | 2,4,4,6,6-Pentamethylhepten-1 sowie |
| 14 Gew.% | an weiterenVerbindungen. |

**Schwefelwasserstoff** wird kontinuierlich durch Umsetzung einer wässrigen Lösung (39-40 Gew. %) von Natriumhydrogensulfid (Lieferant: Carbosulf Akzo Nobel) mit Schwefelsäure (70 %ig) bei einer Temperatur von 55-65°C und einem Druck von 2 bar in einer Menge von 200 kg/h hergestellt. Mitgerissene Schwefelsäure wird in einem Strahlwäscher mit 5%iger Natronlauge ausgewaschen. Vor der Verflüssigung des Schwefelwasserstoffs wird Wasser durch Abkühlen auf 15 °C auskondensiert und durch Abkühlung auf -20°C ausgefroren.
**Bortrifluorid:** gasförmig; Lieferant: BASF/Arkema,; Reinheitsgrad: > 99,5%.

### A KONTINUIERLICHE HERSTELLUNG DES ERFINDUNGSGEMÄßEN GEMISCHS

### Beispiel 1:

Der wie oben beschrieben hergestellte und getrocknete Schwefelwasserstoff wurde mit auf -20 °C vorgekühltem TIB im Molverhältnis 2,71:1 gemischt, auf 3 bar komprimiert und in 2 Stufen auf - 10°C und -30°C gekühlt und verflüssigt.

Anschließend wurde Bortrifluorid in einer Menge von 0,64 Gew. % bezogen auf das eingesetzte TIB bei einem Druck von 8 bar in die auf -30°C gekühlte Mischung von TIB und Schwefelwasserstoff geleitet. Die Reaktion fand in zwei hintereinander geschalteten Rohrreaktoren statt. Die Verwelzeit im ersten Rohrreaktor (Volumen: 111; Längen/Durchmesser-Verhältnis: 9/1) betrug ca. 1 min. Im Anschluss daran wurde die Reaktionsmischung in einen mit Sole auf-60°C im Gegenstromverfahren gekühlten Schlangenrohrreaktor (volumen: 16 1; Längen/Durchmesser-Verhältnis: 166/1; Verweilzeit: ca. 1,5 min) geleitet. Die Eintrittstemperatur des Reaktionsgemisches betrug -15 ± 1°C, die Temperatur beim Austritt am Ende des Schlangenrohrs - 45 ± 2°C.

Zur Abstoppung wurde das Reaktionsgemisch über ein Tauchrohr in ein Abstoppgefäß geleitet, dem kontinuierlich eine wässrige Natriumhydroxidlösung (5 %) zugeführt wurde. Hierdurch wurde der Katalysator zerstört und als Natriumborat und Natriumfluorid ausgewaschen. Durch Einleiten von Wasserdampf wurde die Temperatur im Abstoppgefaß bei 50°C gehalten und gleichzeitig nicht umgesetzter Schwefelwasserstoff ausgetrieben.

Das abgestoppte Reaktionsgemisch wurde kontinuierlich in einen Separator geleitet, in dem die organische Phase des Roh-Mercaptans (Roh-TDDM) als obere Phase von der wässrigen Phase bei 40°C getrennt wurde. Vor der Destillation wurde das Roh-TDDM mit entionisiertem Wasser gewaschen. Zur Entfernung von Restmengen an Schwefelwasserstoff wurde das Roh-Mercaptan bei 65°C / 80 mbar behandelt.

Die Abtrennung der gewünschten Rein-Mercaptanfraktion von hochmolekularen Hochsiedern und von nicht umgesetztem TIB erfolgte durch eine kontinuierliche fraktionierende Destillation unter reduziertem Druck in einer mit Raschigringen gefüllten Kolonne. Im kontinuierlichen Betrieb wurde bei einer Sumpftemperatur von 128-132°C bei 45 mbar sowie einer Kopftemperatur von 74-76°C/ 40 mbar in einem Seitenstrom Rein-Mercaptan mit einer Dichte von 0,8625 g/cm³ abgenommen.

Im 1. Reaktionsdurchgang wurde TIB zu 85 % umgesetzt.

Das nicht umgesetzte TIB wurde gesammelt und in einem 2. Durchgang erneut mit Schwefelwasserstoff umgesetzt. Im zweiten Reaktionsdurchgang wurden 63 % des TIB umgesetzt.

In Summe resultierte somit ein Triisobutenumsatz von 94,5%.

Nach zwei Reaktionsdurchgängen betrug die Ausbeute an destillativ gereinigtem Mercaptangemisch 94,5 % bezogen auf umgesetztes TIB. Die Zusammensetzung des erhaltenen Gemischs ist in Tabelle 1 (Nr.1) dargestellt.

### Beispiele 2-6:

Die Durchführung in den Beispielen 2 bis 6 erfolgte analog zu Beispiel 1, es wurden jedoch unterschiedliche TIB-Chargen eingesetzt, die sich im Wassergehalt unterschieden. Der Wassergehalt ist in Tabelle 1 angegeben, ebenso wie die erhaltenen Ergebnisse.

In der nachfolgenden Tabelle werden die einzelnen Bestandteile des erhaltenen erfindungsgemäßen Gemischs wie folgt abgekürzt:

| | | |
|---|---|---|
| - | 2,2,4,6,6-Pentamethylheptanthiol-4: | Isomer 1 |
| - | 2,4,4,6,6-Pentamethylheptanthiol-2: | Isomer 2 |
| - | 2,3,4,6,6-Pentamethylheptanthiol-2: | Isomer 3 |
| - | 2,3,4,6,6-Pentamethylheptanthiol-3: | Isomer 4 und 4', da es sich um zwei |
| | | Diastereomere handelt. |

**Tabelle 1: Ergebnisse der Beispiele 1-6**

| **Bsp.** | **Wassergehalt des eingesetzten TIB [ppm]** | **TIB-Umsatz (nach 1. Reaktionszyklus) [%]** | **TIB-Umsatz (nach zwei Reaktions-Zyklen) [%]** | **Gesamtausbeute (nach zwei Reaktionszyklen) [%]** | **Isomer 1** | **Isomer 2** | **Isomer 3** | **Isomer 4** | **Isomer 4'** | **weitere Bestandteile** |
|---|---|---|---|---|---|---|---|---|---|---|
| | | | | | **[%]** | **[%]** | **[%]** | **[%]** | **[%]** | **[%]** |
| 1 | 37 | 85 | 94,5 | 95 | 80,0 | 5,3 | 3,8 | 2,6 | 1,9 | 6,4 |
| 2 | 32 | 80 | 93 | 92 | 80,4 | 5,4 | 3,8 | 3,3 | 1,4 | 5,7 |
| 3 | 30 | 82 | 93,4 | 93 | 80,1 | 5,4 | 3,8 | 3,3 | 1,2 | 6,2 |
| 4 | 38 | 81 | 93,4 | 93 | 79,2 | 5,7 | 4,0 | 3,5 | 0,8 | 6,8 |
| 5 | 35 | 80 | 92,6 | 92 | 81,6 | 5,0 | 4,1 | 3,0 | 1,1 | 5,2 |
| 6 | 29 | 83 | 93,7 | 92 | 79,4 | 5,4 | 4,2 | 3,4 | 1,4 | 6,2 |

Aus Tabelle 1 lässt sich erkennen, dass die Zusammensetzung der erfindungsgemäßen Mercaptanmischung, die nach dem 1. und 2. Reaktionsdurchgang erhalten wird, bei Wiederholungen mit verschiedenen Triisobutenchargen innerhalb sehr enger Bandbreiten variiert. Dies ist ein erheblicher Vorteil.

### Gaschromatographische Trennung des erfindungsgemäßen Isomerengemischs und massenspektroskopische Strukturaufklärung der Isomere:

Die Zusammensetzung der in den Beispielen 1-6 erhaltenen Isomerengemische erfolgte mittels Gaschromatographie mit FID-Detektion. Für die Analytik wurden jeweils 50 µl der aus den Beispielen 1-6 erhaltenen Reaktionsprodukte mit 5 mL Toluol verdünnt, wobei jeweils 0,2 µL dieser Lösung gaschromatographisch untersucht wurden. Für die Durchführung der Analysen wurden folgende Bedingungen angewandt:

| | |
|---|---|
| Gaschromatograph: | Fison Trace GC |
| Trennsäule: | Stabilwax-DB (Restek) 30m, 0,25mm ID, 0,25µm df |
| Detektortemperatur: | 260°C |
| Injektortemperatur: | 170°C |
| Ofentemperatur: | 60°C, 2min isotherm Aufheizrate: 5 °Clmin 190°C Endtemperatur, 1min isotherm |
| Trägergas: | Helium, 1,0 mL Säulenfluss |
| Split: | 50 mL/min |

Anschließend wurde die chemische Struktur der mittels Gaschromatographie aufgetrennten Fraktionen des erhaltenen Isomerengemischs durch Massenspektroskopie bestimmt. Dies ist nachfolgend für das Isomerengemisch aus Beispiel 1 dargestellt. Die entsprechenden Massenspektren der einzelnen Fraktionen sind in der folgenden Abbildung dargestellt.

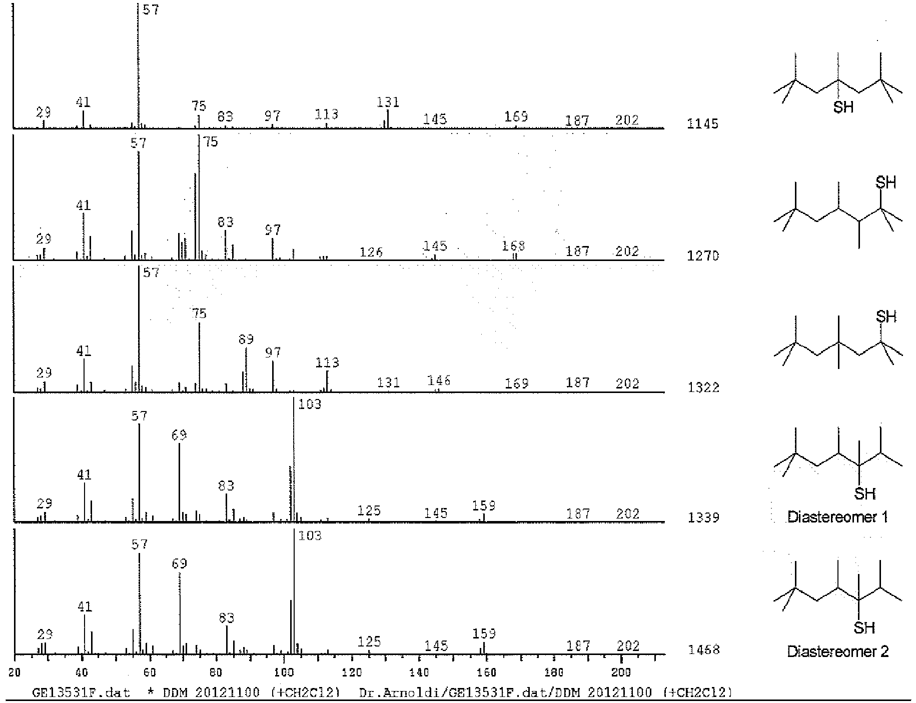

In den Massenspektren treten Peaks mit charakteristischen m/z-Verhältnissen auf:
159 entsteht durch Abspaltung eines Propylradikals (m/z = 43) aus dem radikalischen Molekülion (m/z = 202)
113 entsteht durch Abspaltung eines schwefelhaltigen Radikals (m/z = 89) aus dem radikalischen Molekülion (m/z = 202)
103 entsteht durch Abspaltung eines C₇H₁₅-Radikals (m/z = 99) aus dem radikalischen Molekülion mit mlz 202 oder durch Abspaltung von Isobuten (m/z = 56) aus m/z = 159 97 entsteht durch Abspaltung von H₂S (m/z = 34) aus m/z = 131 bzw. durch Abspaltung eines Ethylradikals (m/z = 29) aus m/z = 126
89 entsteht durch Abspaltung von m/z = 113 aus dem radikalischen Molekülion mit m/z = 202
75 entsteht durch Abspaltung von C₉H₁₉ (m/z = 127) aus dem radikalischen Molekülion (m/z = 202)
69 C₅H₉-Kation, entsteht durch Abspaltung von H₂S (m/z = 34) aus m/z = 103
57 tert.-Butyl-Kation (C₄H₉)
41 Propyl-Kation (C₃H₅).

### Die Zuordnung erfolgte auf der Basis folgender Literaturstellen:

H. Budzikiewicz, C. Djerassi, D.H. Williams, Mass Spectrometry of Organic Compounds, Holden-Day Inc., San Francisco, 1967.
J.H. Gross, Mass Spectrometry, Springer-Verlag, Heidelberg, 2004.

### B HERSTELLUNG VON NITRILKAUTSCHUK UNTER EINSATZ DES ERFINDUNGSGEMÄßEN ISOMERENGEMISCHS

Für die Herstellung des Nitrilkautschuks wurden die in Tabelle 2 aufgeführten Einsatzstoffe verwendet, wobei sämtliche Rezepturbestandteile in Gew.-Teilen angegeben sind und auf 100 Gew.-Teile der Monomermischung bezogen werden. In Tabelle 2 sind auch die übrigen Polymerisationsparameter genannt.

**Tabelle 2:**

| | **Erfindungsgemäße Beispiele** | | |
|---|---|---|---|
| | **7** | **8** | **9** |
| Butadien (Gew.-Teile) | 73 | 73 | 73 |
| Acrylnitril (Gew.-Teile) | 27 | 27 | 27 |
| Gesamt-Wassermenge | 200 | 200 | 200 |
| Erkantol^{®} BXG¹⁾ | 3,69 | 3,69 | 3,69 |
| Baykanol^{®} PQ²⁾ | 1,10 | 1,10 | 1,10 |
| K-Salz der Kokosfettsäure | 0,72 | 0,72 | 0,72 |
| KOH | 0,05 | 0,05 | 0,05 |
| erfindungsgemäßes Gemisch aus Beispiel 1 | 0,21^{3a)}/0,21^{3b)} | 0,23^{3a)}/0,23^{3b)} | 0,25^{3a)}/0,24^{3b)} |
| Kaliumperoxodisulfat | 0,39^{4a)}/0,20^{4b)} | 0,39^{4a)}/0,20^{4b)} | 0,39^{4a)}/0,20^{4b)} |
| Tris-(α-hydroxy-ethyl)-amin | 0,57 | 0,57 | 0,57 |
| Na-Dithionit | 1,28 | 1,28 | 1,28 |
| 2,6 Di-tert.butyl-p-kresol⁵⁾ | 1,25 | 1,25 | 1,25 |
| | | | |
| Polymerisationstemperatur [°C] | 13 | 13 | 13 |
| Polymerisationsumsatz [%] | 66 | 68 | 70 |
| | | | |
| ACN-Gehalt des Nitrilkautschuks [Gew.%] | 27,9 | 27,1 | 26,8 |
| ML (1+4 @ 100°C) des Nitrilkautschuks [ME] | 139 | 82 | 40 |

| | | | |
|---|---|---|---|
| ¹⁾ Natriumsalz einer Mischung von mono- und disulfonierten Naphthalinsulfonsäuren mit Isobutylenoligomerresten (Erkantol^{®} BXG) ²⁾ Natriumsalz von Methylen-bis-Naphthalinssulfonat (Baykanol^{®} PQ, Lanxess Deutschland GmbH) ^{3a)} Bei Polymerisationsbeginn vorgelegte Gew.-Teile ^{3b)} Bei 15% Umsatz nachdosierte Gew.-Teile ^{4a)} Bei Polymerisationsbeginn vorgelegte Gew.-Teile ^{4b)} Bei 15 % Umsatz nachdosierte Gew.-Teile ⁵⁾ Vulkanox^{®} KB; Lanxess Deutschland GmbH | | | |

Die Durchführung der Polymerisationen erfolgte diskontinuierlich in einem 200 1-Autoklaven mit Rührwerk. Bei den Autoklavenansätzen wurden jeweils 35 kg der Monomermischung und eine Gesamtwassermenge von 70 kg verwendet. Von dieser Wassermenge wurden jeweils 60 kg mit den Emulgatoren (Erkantol^{®}BXG, Baykanol^{®}PQ und K-Salz der Kokosfettsäure) und Natriumhydroxid im Autoklaven vorgelegt und mit einem Stickstoffstrom gespült. Mit der Restwassermenge wurden wässrige Lösungen von Kaliumperoxodisulfat, Tris-(α-hydroxy-ethyl)-amin und Natriumdithionit hergestellt. Danach wurden die entstabilisierten Monomeren und die in Tabelle 2) angegebenen Mengen des erfindungsgemäßen Mercaptangemischs aus Beispiel 1 zugegeben und der Reaktor verschlossen. Nach Thermostatisierung des Reaktorinhalts wurden die Polymerisationen durch sukzessive Zugabe wässriger Lösungen von Tris-(α-hydroxy-ethyl)-amin und von Kaliumperoxodisulfat (in den jeweils angegebenen Teilmengen) gestartet. Der Polymerisationsverlauf wurde durch gravimetrische Umsatzbestimmung verfolgt. Bei einem Polymerisationsumsatz von 15 % wurden die Restmengen an erfindungsgemäßem Gemisch aus Beispiel 1 sowie an Kaliumperoxodisulfat nachdosiert. Bei Erreichen von ca. 65-70 % Umsatz wurden die Polymerisation durch Zugabe einer wässrigen Lösung von Natriumdithionit abgestoppt und mit einer Lösung von Vulkanox^{®} KB, gelöst in kleiner Menge an Acrylnitril, versetzt. Nicht umgesetzte Monomere und sonstige flüchtige Bestandteile wurden mittels Wasserdampfdestillation entfernt.

Zur Bestimmung der Mooney-Viskosität wurden aliquote Teile der Latices mit einer Calciumchloridlösung koaguliert. Hierfür wurden jeweils 3 Gew. % Calciumchlorid bezogen auf Nitrilkautschuk verwendet. Die Koagulation erfolgte in einem rührbaren Behälter, wobei die wässrige Calciumchloridlösung (0,03 Gew.-%ig) in dem Behälter vorgelegt und auf 70°C erwärmt wurde. Der Latex wurde langsam unter Rühren in die Fällmittellösung gegeben. Nach der Koagulation wurden die Kautschukkrümel mittels eines Siebes entfernt und durch zweimalige Redispergierung mit Wasser bei 70°C gewaschen, durch Auspressen auf eine Restfeuchte von 5 bis 20 Gew. % vorentwässert und diskontinuierlich in einem Umlufttrockenschrank auf eine Restfeuchte von < 0, 6 % getrocknet.

Die Mooney-Viskosität (ML 1+4@100°C) wurde mittels eines Scherscheibenviskosimeters nach DIN 53523/3 bzw. ASTM D 1646 bei 100°C bestimmt.

Mit dieser Versuchsserie wird gezeigt, dass bei Verwendung des erfindungsgemäßen Mercaptangemischs die gezielte Einstellung von Mooney-Viskositäten im Bereich von z.B. 40 bis 140 Mooneyeinheiten möglich ist und dass für die Molekulargewichtseinstellung nur geringe Mengen des Mercaptangemischs notwendig sind.

## Patentansprüche

1. Verfahren zur Herstellung eines Gemischs enthaltend
2,2,4,6,6-Pentamethylheptanthiol-4,
2,4,4,6,6-Pentamethylheptanthiol-2,
2,3,4,6,6-Pentamethylheptanthiol-2 und 2,3,4,6,6-Pentamethylheptanthiol-3,
umfassend die Umsetzung von Schwefelwasserstoff mit Trüsobuten in einem kontinuierlichen Verfahren bei Temperaturen von 0°C bis -60°C, wobei
(a) der Schwefelwasserstoff vor der Umsetzung einer Trocknung unterzogen wird,
(b) das eingesetzte Trüsobuten einen Wassergehalt von 0 bis 70 ppm besitzt,
(c) als Katalysator Bortrifluorid in Mengen von 0,25 bis 1,2 Gew.%, bezogen auf das eingesetzte Trüsobuten verwendet wird,
(d) die Umsetzung in Abwesenheit von mit Bortrifluorid komplexbildenden Verbindungen erfolgt und
(e) das Reaktionsgemisch im Anschluss an die Umsetzung mit einer wässrigen alkalischen Lösung enthaltend Ammoniak, Amine, Hydroxide, Hydrogencarbonate oder Carbonate in gelöster Form in Kontakt gebracht und der Katalysator entfernt wird,
wobei das Triisobuten
| | | |
|---|---|---|
| 45 bis 55 | Gew.% | 2,2,4,6,6-Pentamethylhepten-3, |
| 30 bis 40 | Gew.% | 2-(2,2-Dimethylpropyl)-4,4-dimethylpenten-1, |
| 1 bis 5 | Gew.% | 2,4,4,6,6-Pentamethylhepten-2, |
| 1 bis 5 | Gew.% | 2,4,4,6,6-Pentamethylhepten-1 und |
| 5 bis 15 | Gew.% | weitere Verbindungen |
enthält, wobei sich alle vorgenannten Komponenten zu 100 Gew.% aufsummieren.

2. Verfahren nach Anspruch 1, wobei das Trüsobuten einen Wassergehalt von 0 bis 50 ppm besitzt.

3. Verfahren nach Anspruch 1 oder 2, wobei der eingesetzte Schwefelwasserstoff vor der Umsetzung einer Trocknung unterzogen wird, indem in einer 1. Stufe das im Schwefelwasserstoff enthaltene Wasser durch Abkühlung auf eine Temperatur im Bereich von -10°C bis 30°C, bevorzugt im Bereich von 0°C bis 20°C, besonders bevorzugt im Bereich von 3 °C bis 18 °C und insbesondere im Bereich von 10°C bis 15°C auskondensiert und in der 2. Stufe der Schwefelwasserstoff erneut auf eine Temperatur im Bereich von -15°C bis -50°C, bevorzugt im Bereich von -10°C bis 25 °C abgekühlt wird.

4. Verfahren nach einem oder mehreren der Ansprüche 1 bis 3, wobei vor der Zugabe des Katalysators der Schwefelwasserstoff in flüssiger Form mit dem Trüsobuten gemischt und gekühlt wird.

5. Verfahren nach einem oder mehreren der Ansprüche 1 bis 4, wobei das Molverhältnis von Schwefelwasserstoff zu Trüsobuten im Bereich von (1,1-10,0):1, bevorzugt im Bereich von (1,1-5,0):1, besonders bevorzugt im Bereich (1,1-3,0):1 und insbesondere im Bereich von (1,2-2,8):1 liegt.

6. Verfahren nach Anspruch 4 oder 5, wobei die Temperatur der Mischung von Schwefelwasserstoff und Trüsobuten vor der Zugabe des Katalysators im Bereich von -50°C bis 0°C, bevorzugt im Bereich von - 40°C bis -30°C und besonders bevorzugt im Bereich von -35 bis -25 °C liegt.

7. Verfahren nach einem oder mehreren der Ansprüche 1 bis 6, wobei die Dosierung des Bortrifluorids gasförmig bei einem Überdruck im Bereich von 5 bis 10 bar, vorzugsweise im Bereich von 7 bis 8 bar erfolgt.

8. Verfahren nach einem oder mehreren der Ansprüche 1 bis 7, wobei die Menge an Bortrifluorid bezogen auf das eingesetzte Trüsobuten 0,5 bis 1,0 Gew. %, insbesondere 0,6 bis 0,9 Gew.% und insbesondere bevorzugt 0,65 bis 0,85 Gew. % beträgt.

9. Verfahren nach einem oder mehreren der Ansprüche 1 bis 8, wobei die Durchführung in einem oder in zwei hintereinander geschalteten Rohrreaktoren erfolgt.

10. Verfahren nach einem oder mehreren der Ansprüche 1 bis 8, wobei die Durchführung in zwei hintereinander geschaltete Rohrreaktoren erfolgt und die Verweilzeit im ersten Reaktor 0,5 bis 5 min, vorzugsweise 1 bis 3 min und im zweiten Reaktor 0,5 bis 5 min, vorzugsweise 1 bis 3 min beträgt.

11. Verfahren nach Anspruch 10, wobei die Eintrittstemperatur des Reaktionsgemischs in den zweiten Rohrreaktor im Bereich von 0°C bis -30°C, bevorzugt im Bereich von -5°C bis -25°C und die Austrittstemperatur bei -40°C bis -60°C liegt.

12. Verfahren nach einem oder mehreren der Ansprüche 1 bis 11, wobei das Reaktionsgemisch nach Beendigung der Umsetzung in eine wässrige alkalische Lösung eingeleitet wird.

13. Verfahren nach Anspruch 12, wobei es sich um eine wässrige Lösung handelt, die Natriumhydroxid, Kaliumhydroxid, Natriumcarbonat, Kaliumcarbonat, Natriumhydogencarbonat und Kaliumhydrogencarbonat in gelöster Form enthält.

14. Verfahren nach Anspruch 13, wobei während des Kontakts zwischen Reaktionsgemisch und wässriger alkalischer Lösung die Temperatur im Bereich von 10°C bis 100°C, bevorzugt im Bereich von 30°C bis 90°C und insbesondere im Bereich von 50°C bis 80 °C gehalten wird.

15. Gemisch enthaltend 70-85 Flächen% 2,2,4,6,6-Pentamethylheptanthiol-4, 3-6 Flächen% 2,4,4,6,6-Pentamethylheptanthiol-2, 2-5 Flächen% 2,3,4,6,6-Pentamethylheptanthiol-2, 3-13 Flächen% 2,3,4,6,6-Pentamethylheptanthiol-3 und weitere Verbindungen, wobei die Summe aller vorgenannten Isomere und der weiteren Verbindungen 100 Flächen% ergibt.

16. Gemisch gemäß Anspruch 15, welches erhältlich ist durch das Verfahren gemäß einem oder mehreren der Ansprüche 1 bis 14.

17. Verwendung des Gemischs gemäß Anspruch 15 oder 16 zur Regelung des Molekulargewichts bei Polymerisationen zur Herstellung von Kautschuken, insbesondere von Nitrilkautschuken.

18. Synthetischer Kautschuk, der erhältlich ist durch Polymerisation in Gegenwart des Gemischs gemäß Anspruch 15 oder 16 und der 2,2,4,6,6-Pentamethylheptan-4-thio- und/oder 2,4,4,6,6-Pentamethylheptan-2-thio- und/oder 2,3,4,6,6-Pentamethylheptan-2-thio und/oder 2,3,4,6,6-Pentamethylheptan-3-thio-Endgruppen enthält.

19. Verfahren zur Herstellung eines Nitrilkautschuks durch Polymerisation von mindestens einem α,β-ungesättigten Nitril, mindestens einem konjugierten Dien und gegebenenfalls einem oder mehreren weiteren copolymerisierbaren Monomere in Gegenwart des Gemischs gemäß Anspruch 15 oder 16.

## Claims

1. Process for preparing a mixture containing 2,2,4,6,6-pentamethylheptane-4-thiol, 2,4,4,6,6-pentamethylheptane-2-thiol, 2,3,4,6,6-pentamethylheptane-2-thiol and 2,3,4,6,6-pentamethylheptane-3-thiol,
which comprises reaction of hydrogen sulphide with triisobutene at temperatures of from 0°C to -60°C in a continuous process, wherein
(a) the hydrogen sulphide is subjected to drying before the reaction,
(b) the triisobutene used has a water content of from 0 to 70 ppm,
(c) boron trifluoride is used as catalyst in amounts of from 0.25 to 1.2% by weight, based on the triisobutene used,
(d) the reaction is carried out in the absence of compounds which form complexes with boron trifluoride and
(e) the reaction mixture is brought into contact with an aqueous alkaline solution containing ammonia, amines, hydroxides, hydrogencarbonates or carbonates in dissolved form after the reaction to remove the catalyst, wherein the triisobutene contains
from 45 to 55 % by weight of 2,2,4,6,6-penta-methyl-3-heptene,
from 30 to 40% by weight of 2-(2,2-dimethyl-propyl)-4,4-dimethyl-1-pentene,
from 1 to 5% by weight of 2,4,4,6,6-penta-methyl-2-heptene,
from 1 to 5% by weight of 2,4,4,6,6-penta-methyl-1-heptene and
from 5 to 15% by weight of further compounds, with all the abovementioned components adding up to 100% by weight.

2. Process according to Claim 1, wherein the triisobutene has a water content of from 0 to 50 ppm.

3. Process according to Claim 1 or 2, wherein the hydrogen sulphide used is subjected to drying by, in a 1^{st} stage, the water present in the hydrogen sulphide being condensed out by cooling to a temperature in the range from -10°C to 30°C, preferably in the range from 0°C to 20°C, particularly preferably in the range from 3°C to 18°C and in particular in the range from 10°C to 15°C and, in the 2^{nd} stage, the hydrogen sulphide being cooled again to a temperature in the range from -15°C to -50°C, preferably in the range from -10°C to -25°C, before the reaction.

4. Process according to one or more of Claims 1 to 3, wherein the hydrogen sulphide is mixed in liquid form with the triisobutene and cooled before addition of the catalyst.

5. Process according to one or more of Claims 1 to 4, wherein the molar ratio of hydrogen sulphide to triisobutene is in the range (1.1-10.0):1, preferably in the range (1.1-5.0):1, particularly preferably in the range (1.1-3.0):1 and in particular in the range (1.2-2.8):1.

6. Process according to Claim 4 or 5, wherein the temperature of the mixture of hydrogen sulphide and triisobutene before addition of the catalyst is in the range from -50°C to 0°C, preferably in the range from -40°C to -30°C and particularly preferably in the range from -35 to -25°C.

7. Process according to one or more of Claims 1 to 6, wherein the boron trifluoride is introduced in gaseous form at a gauge pressure in the range from 5 to 10 bar, preferably in the range from 7 to 8 bar.

8. Process according to one or more of Claims 1 to 7, wherein the amount of boron trifluoride based on the triisobutene used is from 0.5 to 1.0% by weight, in particular from 0.6 to 0.9% by weight and very particularly preferably from 0.65 to 0.85% by weight.

9. Process according to one or more of Claims 1 to 8, wherein the reaction is carried out in a tube reactor or in two tube reactors connected in series.

10. Process according to one or more of Claims 1 to 8, wherein the reaction is carried out in two tube reactors connected in series and the residence time in the first reactor is from 0.5 to 5 minutes, preferably from 1 to 3 minutes, and that in the second reactor is from 0.5 to 5 minutes, preferably from 1 to 3 minutes.

11. Process according to Claim 10, wherein the temperature of the reaction mixture on entering the second tube reactor is in the range from 0°C to -30°C, preferably in the range from -5°C to -25°C, and the outlet temperature is from -40°C to -60°C.

12. Process according to one or more of Claims 1 to 11, wherein the reaction mixture is passed into an aqueous alkaline solution after the reaction is complete.

13. Process according to Claim 12, wherein the aqueous alkaline solution is an aqueous solution containing sodium hydroxide, potassium hydroxide, sodium carbonate, potassium carbonate, sodium hydrogencarbonate and potassium hydrogencarbonate in dissolved form.

14. Process according to Claim 13, wherein the temperature is kept in the range from 10°C to 100°C, preferably in the range from 30°C to 90°C and in particular in the range from 50°C to 80°C, during the contact between the reaction mixture and the aqueous alkaline solution.

15. Mixture containing 70-85% by area of 2,2,4,6,6-pentamethylheptane-4-thiol, 3-6% by area of 2,4,4,6,6-pentamethylheptane-2-thiol, 2-5% by area of 2,3,4,6,6-pentamethylheptane-2-thiol, 3-13% by area of 2,3,4,6,6-pentamethylheptane-3-thiol and further compounds, where the sum of all the abovementioned isomers and the further compounds is 100% by area.

16. Mixture according to Claim 15 which can be obtained by the process according to one or more of Claims 1 to 14.

17. Use of the mixture according to Claim 15 or 16 for regulating the molecular weight in polymerizations for producing rubbers, in particular nitrile rubbers.

18. Synthetic rubber which can be obtained by polymerization in the presence of the mixture according to Claim 15 or 16 and contains 2,2,4,6,6-pentamethylheptane-4-thio and/or 2,4,4,6,6-pentamethylheptane-2-thio and/or 2,3,4,6,6-pentamethylheptane-2-thio and/or 2,3,4,6,6-pentamethylheptane-3-thio end groups.

19. Process for producing a nitrile rubber by polymerization of at least one α,β-unsaturated nitrile, at least one conjugated diene and optionally one or more further copolymerizable monomers in the presence of the mixture according to Claim 15 or 16.

## Revendications

1. Procédé de fabrication d'un mélange contenant
du 2,2,4,6,6-pentaméthylheptanethiol-4,
du 2,4,4,6,6-pentaméthylheptanethiol-2,
du 2,3,4,6,6-pentaméthylheptanethiol-2 et
du 2,3,4,6,6-pentaméthylheptanethiol-3,
comprenant la mise en réaction de sulfure d'hydrogène avec du triisobutène dans un procédé continu à des températures de 0 °C à -60 °C, selon lequel
(a) le sulfure d'hydrogène est soumis à un séchage avant la réaction,
(b) le triisobutène utilisé présente une teneur en eau de 0 à 70 ppm,
(c) du trifluorure de bore est utilisé en tant que catalyseur en quantités de 0,25 à 1,2 % en poids, par rapport au triisobutène utilisé,
(d) la réaction a lieu en l'absence de composés formant des complexes avec le trifluorure de bore, et
(e) le mélange réactionnel est mis en contact après la réaction avec une solution alcaline aqueuse contenant de l'ammoniac, des amines, des hydroxydes, des hydrogénocarbonates ou des carbonates sous forme dissoute, et le catalyseur est éliminé,
le triisobutène contenant
45 à 55 % en poids de 2,2,4,6,6-pentaméthylheptène-3,
30 à 40 % en poids de 2-(2,2-diméthylpropyl)-4,4-diméthylpentène-1,
1 à 5 % en poids de 2,4,4,6,6-pentaméthylheptène-2, 1 à 5 % en poids de 2,4,4,6,6-pentaméthylheptène-1 et
5 à 15 % en poids d'autres composés,
la somme de tous les composants susmentionnés étant de 100 % en poids.

2. Procédé selon la revendication 1, dans lequel le triisobutène présente une teneur en eau de 0 à 50 ppm.

3. Procédé selon la revendication 1 ou 2, dans lequel le sulfure d'hydrogène utilisé est soumis à un séchage avant la réaction, selon lequel l'eau contenue dans le sulfure d'hydrogène est éliminée par condensation lors d'une 1^{e} étape par refroidissement à une température dans la plage allant de -10 °C à 30 °C, de préférence dans la plage allant de 0 °C à 20 °C, de manière particulièrement préférée dans la plage allant de 3 °C à 18 °C, et notamment dans la plage allant de 10 °C à 15 °C, et, lors de la 2^{e} étape, le sulfure d'hydrogène est de nouveau refroidi à une température dans la plage allant de -15 °C à -50 °C, de préférence dans la plage allant de -10 °C à 25 °C.

4. Procédé selon une ou plusieurs des revendications 1 à 3, dans lequel le sulfure d'hydrogène est mélangé sous forme liquide avec le triisobutène et refroidi avant l'ajout du catalyseur.

5. Procédé selon une ou plusieurs des revendications 1 à 4, dans lequel le rapport molaire entre le sulfure d'hydrogène et le triisobutène se situe dans la plage allant de (1,1 à 10,0):1, de préférence dans la plage allant de (1,1 à 5,0):1, de manière particulièrement préférée dans la plage allant de (1,1 à 3,0):1, et notamment dans la plage allant de (1,2 à 2,8):1.

6. Procédé selon la revendication 4 ou 5, dans lequel la température du mélange de sulfure d'hydrogène et de triisobutène avant l'ajout du catalyseur se situe dans la plage allant de -50 °C à 0 °C, de préférence dans la plage allant de -40 °C à -30 °C et de manière particulièrement préférée dans la plage allant de -35 à -25 °C.

7. Procédé selon une ou plusieurs des revendications 1 à 6, dans lequel l'introduction du trifluorure de bore a lieu sous forme gazeuse à une surpression dans la plage allant de 5 à 10 bar, de préférence dans la plage allant de 7 à 8 bar.

8. Procédé selon une ou plusieurs des revendications 1 à 7, dans lequel la quantité de trifluorure de bore par rapport au triisobutène utilisé est de 0,5 à 1,0 % en poids, notamment de 0,6 à 0,9 % en poids, et de manière particulièrement préféré de 0,65 à 0,85 % en poids.

9. Procédé selon une ou plusieurs des revendications 1 à 8, dans lequel la réalisation a lieu dans un ou dans deux réacteurs tubulaires connectés en série.

10. Procédé selon une ou plusieurs des revendications 1 à 8, dans lequel la réalisation a lieu dans deux réacteurs tubulaires connectés en série, et le temps de séjour dans le premier réacteur est de 0,5 à 5 min, de préférence de 1 à 3 min et dans le second réacteur de 0,5 à 5 min, de préférence de 1 à 3 min.

11. Procédé selon la revendication 10, dans lequel la température d'entrée du mélange réactionnel dans le second réacteur tubulaire se situe dans la plage allant de 0 °C à -30 °C, de préférence dans la plage allant de -5 °C à -25 °C, et la température de sortie de -40 °C à -60 °C.

12. Procédé selon une ou plusieurs des revendications 1 à 11, dans lequel le mélange réactionnel est conduit dans une solution alcaline aqueuse après la fin de la réaction.

13. Procédé selon la revendication 12, dans lequel il s'agit d'une solution aqueuse qui contient de l'hydroxyde de sodium, de l'hydroxyde de potassium, du carbonate de sodium, du carbonate de potassium, de l'hydrogénocarbonate de sodium et de l'hydrogénocarbonate de potassium sous forme dissoute.

14. Procédé selon la revendication 13, dans lequel, pendant le contact entre le mélange réactionnel et la solution alcaline aqueuse, la température est maintenue dans la plage allant de 10 °C à 100 °C, de préférence dans la plage allant de 30 °C à 90 °C, et notamment dans la plage allant de 50 °C à 80 °C.

15. Mélange contenant 70 à 85 % en surface de 2,2,4,6,6-pentaméthylheptanethiol-4, 3 à 6 % en surface de 2,4,4,6,6-pentaméthylheptanethiol-2, 2 à 5 % en surface de 2,3,4,6,6-pentaméthylheptanethiol-2, 3 à 13 % en surface de 2,3,4,6,6-pentaméthylheptanethiol-3 et d'autres composés, la somme de tous les isomères susmentionnés et des autres composés étant de 100 % en surface.

16. Mélange selon la revendication 15, qui peut être obtenu par le procédé selon une ou plusieurs des revendications 1 à 14.

17. Utilisation du mélange selon la revendication 15 ou 16 pour la régulation du poids moléculaire lors de polymérisations pour la fabrication de caoutchoucs, notamment de caoutchoucs de nitrile.

18. Caoutchouc synthétique, qui peut être obtenu par polymérisation en présence du mélange selon la revendication 15 ou 16 et des groupes terminaux 2,2,4,6,6-pentaméthylheptane-4-thio et/ou 2,4,4,6,6-pentaméthylheptane-2-thio et/ou 2,3,4,6,6-pentaméthylheptane-2-thio et/ou 2,3,4,6,6-pentaméthylheptane-3-thio.

19. Procédé de fabrication d'un caoutchouc de nitrile par polymérisation d'au moins un nitrile α,β-insaturé, d'au moins un diène conjugué et éventuellement d'un ou de plusieurs autres monomères copolymérisables en présence du mélange selon la revendication 15 ou 16.
